# EUROPEAN PATENT APPLICATION

(11) **EP 4 717 764 A1**
(43) Date of publication of application: **01.04.2026**
(21) Application number: 25823104.2
(22) Date of filing: 13.05.2025
(51) Int. Cl.: C12N 1/20, A61K 35/747, A61K 35/745, A61K 35/742, A61P 1/10, C12R 1/225, C12R 1/07

(54) **COMPOSITE PROBIOTIC FOR RELIEVING CONSTIPATION, AND USE THEREOF**

(30) Priority: 02.07.2024 CN 202410874628
(71) Applicant: Wecare Probiotics Co., Ltd., Suzhou, Jiangsu 215200 (CN)
(72) Inventor: FANG, Shuguang, Suzhou, Jiangsu 215200 (CN); QI, Yongmei, Suzhou, Jiangsu 215200 (CN); DONG, Yao, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Prinz & Partner mbB
(86) International application number: PCT/CN2025/094546
(87) International publication number: WO 2026/007549

(57) **Abstract**

Provided are a composite probiotic for improving constipation and a use thereof. The composite probiotic for improving constipation is composed of a *Bifidobacterium animalis subsp. lactis* BLa36 strain deposited under CGMCC No. 24029, a *Lactobacillus rhamnosus* LRa05 strain deposited under CGMCC No. 24377, a *Bacillus coagulans* BC99 strain deposited under CGMCC No. 19487, and a *Bifidobacterium bifidum* BBi32 strain deposited under CGMCC No. 16923. The four strains have a synergistic effect in relieving constipation symptoms.

## Description

### TECHNICAL FIELD

The present application belongs to the technical field of probiotics and relates to a composite probiotic for improving constipation and a use thereof.

### BACKGROUND

Constipation is a common health problem that can be caused by a variety of factors including an improper diet, life stress, and side effects of drugs. Constipation causes discomfort and pain to people, severely affects the quality of life of people, and may also trigger other health problems such as hemorrhoids and intestinal diseases. Constipation is usually caused by slow intestinal peristalsis or insufficient water and is manifested as persistent difficulty in defecation, infrequent bowel movements, dry stools, or incomplete defecation. Frequent or chronic constipation may induce metabolic disorders and other related diseases, such as ischemic stroke, irritable bowel syndrome, and coronary heart disease, with higher risks in elderly patients.

Probiotics are living microorganisms that, when administered in a sufficient amount, are beneficial to the health of a host. Probiotics can alleviate constipation through various mechanisms, including regulating the gut microbiota and fermentation and modulating the nervous system and immune system. Constipation often leads to a microecological imbalance in the intestines, resulting in an increased number of harmful bacteria and a decreased number of probiotics. Probiotics have the functions of maintaining a balance in the gut microbiota, promoting intestinal peristalsis, and improving constipation. Moreover, probiotics in the human colon can digest soluble dietary fiber to produce short-chain fatty acids, further promoting intestinal peristalsis and helping solve the constipation problem. Therefore, taking probiotics helps restore the dominant position of probiotics in the intestines, enabling the effective exertion of the above two mechanisms.

Probiotics can also improve a series of problems caused by constipation, such as bloating, diarrhea, loss of appetite, and intestinal indigestion. Studies have shown significant differences in the compositions of the gut microbiota in constipation patients and non-constipation patients. The composition of fecal microorganisms has been proven to be related to the colonic transit time, and the composition of colonic mucosal microorganisms is directly related to constipation symptoms. Experiments have proven that after supplementation with probiotics, intestinal microbial communities undergo significant changes, indicating that the use of probiotics may affect the components of some microbial communities. Therefore, it is crucial to develop a probiotic product that can effectively prevent, alleviate, or treat constipation.

### SUMMARY

The present application provides a composite probiotic for improving constipation and a use thereof.

In a first aspect, the present application provides a composite probiotic for improving constipation. The composite probiotic for improving constipation is composed of a *Bifidobacterium animalis subsp. lactis* BLa36 strain deposited under CGMCC No. 24029, a *Lactobacillus rhamnosus* LRa05 strain deposited under CGMCC No. 24377, a *Bacillus coagulans* BC99 strain deposited under CGMCC No. 19487, and a *Bifidobacterium bifidum* BBi32 strain deposited under CGMCC No. 16923.

The present application develops a new probiotic compounding manner and a new strategy for improving constipation, that is, the *Bifidobacterium animalis subsp. lactis* BLa36 strain, the *Lactobacillus rhamnosus* LRa05 strain, the *Bacillus coagulans* BC99 strain, and the *Bifidobacterium bifidum* BBi32 strain are compounded. It has been found that the four strains have potential interactions and can collaborate with each other and achieve a synergistic effect. Compared with a strain intervention manner lacking in any strain under the same bacterial dosage, the compounding of the four strains yields significantly improved effects in promoting intestinal peristalsis and relieving constipation symptoms, which are specifically manifested as (1) significantly increasing a level of gastrointestinal peptides such as motilin, substance P, and vasoactive intestinal peptide; (2) significantly increasing an intestinal propulsion rate; and (3) significantly increasing a short-chain fatty acid level in the gastrointestinal tract. Therefore, the composite probiotic has a good prospect of being used for preparing a drug for preventing, alleviating, or treating constipation. Meanwhile, the four strains are probiotics and are highly safe and unlikely to cause dependency when used for preparing related functional products.

The composite probiotic is prepared by a conventional technical method in the art. For example, the method may include: activating the four strains, and inoculating the activated four strains in respective media for culture to obtain culture solutions; centrifuging the culture solutions and suspending bacteria to obtain bacterial suspensions; and mixing four bacterial suspensions according to the ratio of viable bacteria counts. Alternatively, a protectant is further added for lyophilization to prepare a lyophilized probiotic powder product.

Preferably, each of the media includes an MRS medium.

Preferably, the MRS medium includes, by concentration, peptone at 8-12 g/L, beef extract at 8-12 g/L, glucose at 15-25 g/L, sodium acetate at 1-3 g/L, yeast extract at 3-7 g/L, diammonium hydrogen citrate at 1-3 g/L, K₂PO₄·3H₂O at 2-3 g/L, MgSO₄·7H₂O at 0.05-0.2 g/L, MnSO₄ at 0.01-0.1 g/L, Tween 80 at 0.5-2 mL/L, and L-cysteine hydrochloride at 0.1-1 g/L.

Preferably, a ratio of a viable bacteria count of the BLa36 strain, a viable bacteria count of the LRa05 strain, a viable bacteria count of the BC99 strain, and a viable bacteria count of the BBi32 strain is (1-10):(1-10):(1-10):(1-10).

"1-10" may include, for example, 1, 1.5, 2, 2.5, 3, 3.5, 4, 4.5, 5, 5.5, 6, 6.5, 7, 7.5, 8, 8.5, 9, 9.5, and 10. Other specific point values within this numerical range may be selected and are not exhausted here.

Based on the potential interaction relationship between the four strains, the present application has also found that when compounded at the particular ratio of viable bacteria counts, the four strains have more significant efficacy in promoting intestinal peristalsis and relieving constipation symptoms.

In a second aspect, the present application provides a probiotic agent for improving constipation. The probiotic agent includes the composite probiotic of the first aspect.

Preferably, a viable bacteria count of each of the BLa36 strain, the LRa05 strain, the BC99 strain, and the BBi32 strain in the probiotic agent is not less than 1×10⁸ CFU/mL or 1×10⁸ CFU/g, for example, 1×10⁸ CFU/mL (CFU/g), 2×10⁸ CFU/mL (CFU/g), 5×10⁸ CFU/mL (CFU/g), 8×10⁸ CFU/mL (CFU/g), 1×10⁹ CFU/mL (CFU/g), 5×10⁹ CFU/mL (CFU/g), or 1×10¹⁰ CFU/mL (CFU/g). Other specific point values within this numerical range may be selected and are not exhausted here.

Preferably, a dosage form of the probiotic agent includes lyophilized powder, a capsule, a tablet, or a granule.

Preferably, the probiotic agent further includes a lyoprotectant and/or an auxiliary additive.

Preferably, the lyoprotectant includes any one or a combination of at least two of skimmed milk, sucrose, lactose, trehalose, dextran, gelatin, dextrin, arabic gum, sodium alginate, polyvinylpyrrolidone, sorbitol, or xylitol.

Preferably, the auxiliary additive includes any one or a combination of at least two of fructooligosaccharides, xylooligosaccharides, galactooligosaccharides, isomaltooligosaccharides, soybean oligosaccharides, Krestin, polydextrose, α-whey protein, or lactoferrin.

In a third aspect, the present application provides a use of the composite probiotic of the first aspect or the probiotic agent of the second aspect for preparing a drug for preventing, alleviating, or treating constipation.

In a fourth aspect, the present application provides a use of the composite probiotic of the first aspect or the probiotic agent of the second aspect for preparing a drug for increasing a gastrointestinal peptide level.

In a fifth aspect, the present application provides a use of the composite probiotic of the first aspect or the probiotic agent of the second aspect for preparing a drug for increasing a short-chain fatty acid level in the gastrointestinal tract.

Preferably, the drug further includes a pharmaceutically acceptable adjuvant.

Preferably, the adjuvant includes any one or a combination of at least two of a filler, a pH adjusting agent, an anti-oxidant, a binder, a wetting agent, a disintegrating agent, an emulsifier, a cosolvent, a solubilizer, an osmotic pressure regulator, a colorant, a bacteriostatic agent, or a buffer.

Compared with the existing art, the present application has the beneficial effects below.

The present application develops a new probiotic compounding manner and a new strategy for improving constipation, that is, the *Bifidobacterium animalis subsp. lactis* BLa36 strain, the *Lactobacillus rhamnosus* LRa05 strain, the *Bacillus coagulans* BC99 strain, and the *Bifidobacterium bifidum* BBi32 strain are compounded. It has been found that the four strains have potential interactions and can collaborate with each other and achieve a synergistic effect. Compared with a strain intervention manner lacking in any strain under the same bacterial dosage, the compounding of the four strains yields significantly improved effects in promoting intestinal peristalsis and relieving constipation symptoms, which are specifically manifested as (1) significantly increasing a level of gastrointestinal peptides such as motilin, substance P, and vasoactive intestinal peptide; (2) significantly increasing an intestinal propulsion rate; and (3) significantly increasing a short-chain fatty acid level in the gastrointestinal tract. Therefore, the composite probiotic has a good prospect of being used for preparing the drug for preventing, alleviating, or treating constipation. Meanwhile, the four strains are probiotics and are highly safe and unlikely to cause dependency when used for preparing the related functional products.

The taxonomic name of the *Bifidobacterium animalis subsp. lactis* BLa36 strain involved in the present application is *Bifidobacterium animalis subsp. Lactis.* The *Bifidobacterium animalis subsp. lactis* BLa36 strain is deposited under CGMCC No. 24029 on December 2, 2021 in the China General Microbiological Culture Collection Center located at Room 3, No. 1 West Beichen Road, Chaoyang District, Beijing.

The taxonomic name of the *Lactobacillus rhamnosus* LRa05 strain involved in the present application is *Lactobacillus rhamnosus.* The *Lactobacillus rhamnosus* LRa05 strain is deposited under CGMCC No. 24377 on January 24, 2022 in the China General Microbiological Culture Collection Center located at Room 3, No. 1 West Beichen Road, Chaoyang District, Beijing.

The taxonomic name of the *Bacillus coagulans* BC99 strain involved in the present application is *Bacillus coagulans.* The *Bacillus coagulans* BC99 strain is deposited under CGMCC No. 19487 on March 18, 2020 in the China General Microbiological Culture Collection Center located at Room 3, No. 1 West Beichen Road, Chaoyang District, Beijing.

The taxonomic name of the *Bifidobacterium bifidum* BBi32 strain involved in the present application is *Bifidobacterium bifidum.* The *Bifidobacterium bifidum* BBi32 strain is deposited under CGMCC No. 16923 on December 10, 2018 in the China General Microbiological Culture Collection Center located at Room 3, No. 1 West Beichen Road, Chaoyang District, Beijing.

### BRIEF DESCRIPTION OF DRAWINGS

FIG. 1 is a graph showing the statistics on body weights of various groups of mice.
FIG. 2 is a graph showing the statistics on fecal moisture contents of various groups of mice.
FIG. 3 is a graph showing the statistics on intestinal propulsion rates of various groups of mice.
FIG. 4 is a graph showing the statistics on first black stool defecation of various groups of mice.
FIG. 5 is a graph showing the statistics on intestinal acetic acid contents of various groups of mice.
FIG. 6 is a graph showing the statistics on intestinal butyric acid contents of various groups of mice.
FIG. 7 is a graph showing the statistics on motilin contents in serum of various groups of mice.
FIG. 8 is a graph showing the statistics on substance P contents in serum of various groups of mice.
FIG. 9 is a graph showing the statistics on vasoactive intestinal peptide contents in serum of various groups of mice.

### DETAILED DESCRIPTION

Technical solutions of the present application are further described below through examples. It is to be understood by those skilled in the art that the examples are intended to facilitate understanding of the present application and are not to be construed as limiting the present application.

The taxonomic name of the BLa36 strain involved in the following examples is *Bifidobacterium animalis subsp. Lactis.* The BLa36 strain is deposited under CGMCC No. 24029.

The taxonomic name of the LRa05 strain involved in the following examples is *Lactobacillus rhamnosus.* The LRa05 strain is deposited under CGMCC No. 24377.

The taxonomic name of the BC99 strain involved in the following examples is *Bacillus coagulans.* The BC99 strain is deposited under CGMCC No. 19487.

The taxonomic name of the BBi32 strain involved in the following examples is *Bifidobacterium bifidum.* The BBi32 strain is deposited under CGMCC No. 16923.

The formulation of the medium involved in the following examples is as follows:
peptone 10 g/L, beef extract 10 g/L, glucose 20 g/L, sodium acetate 2 g/L, yeast extract 5 g/L, diammonium hydrogen citrate 2 g/L, K₂PO₄·3H₂O 2.6 g/L, MgSO₄·7H₂O 0.1 g/L, MnSO₄ 0.05 g/L, polysorbate 80 (Tween 80) 1 mL/L, and L-cysteine hydrochloride 0.5 g/L.

The bacterial suspension involved in the following examples is prepared by the following method: the required strain is inoculated in a liquid medium and cultured for 24 h at 37°C for activation, and two consecutive activations are performed to obtain an activation solution; the activation solution is inoculated in a liquid medium at an inoculum size of 5% (v/v) and cultured for 24 h at 37°C to obtain a bacterial solution; the bacterial solution is centrifuged at 4°C and 5000 rpm for 10 min and filtered to obtain bacterial cells, and the bacterial cells are resuspended with a PBS solution to obtain the bacterial suspension.

Data on test results are statistically analyzed using ggplot2 in language R. Compared with a control group, ### represents p < 0.001, and ## represents p < 0.01; compared with a model group, *** represents p < 0.001, ** represents p < 0.01, * represents p < 0.05, and ns represents no significant difference.

### Example

This example explores an effect of the composite probiotic of the present application in improving constipation symptoms of mice.

### (1) Test animals

Healthy female SPF-grade Kunming mice were provided by Shanghai Laboratory Animal Center, and the mice were fed with a standard pelleted rodent diet and purified water. All procedures involving mice were in accordance with the guidelines provided by Shanghai Laboratory Animal Care and Experimental Center (license No. 2023052101).

### (2) Animal grouping, modeling, and intervention manners

After two weeks of adaptive feeding, 54 mice were randomly divided into 9 groups, which were a control group (CTL), a model group (MG), test group 1 (BLa36+LRa05+BC99, where the ratio of viable bacteria counts was 5:1:1), test group 2 (BLa36+LRa05+BBi32, where the ratio of viable bacteria counts was 5:1:1), test group 3 (BLa36+BC99+BBi32, where the ratio of viable bacteria counts was 5:1:1), test group 4 (LRa05+BC99+BBi32, where the ratio of viable bacteria counts was 1:1:1), test group 5 (BLa36+LRa05+BC99+BBi32, where the ratio of viable bacteria counts was 5:1:1:1), test group 6 (*Bifidobacterium animalis subsp. lactis* ATCC700541+LRa05+BC99+BBi32, where the ratio of viable bacteria counts was 5:1:1:1), and test group 7 (BLa36+LRa05+BC99+*Bacillus coagulans* ATCC7050, where the ratio of viable bacteria counts was 5:1:1:1), respectively. In each of test groups 1 to 9, the total amount of bacterial suspension was 1×10⁹ CFU/day/mouse.

All the other groups than the control group were orally administered with loperamide (10 mg/kg) once a day for 14 consecutive days to induce the establishment of mouse constipation models. The bacterial suspension of composite probiotic was suspended in water. Test groups 1 to 9 were administered by gavage once a day on Day 15 to Day 28, and the control group and the model group were administered by gavage with normal saline (10 mL/kg).

### (3) Analysis of body weights of mice

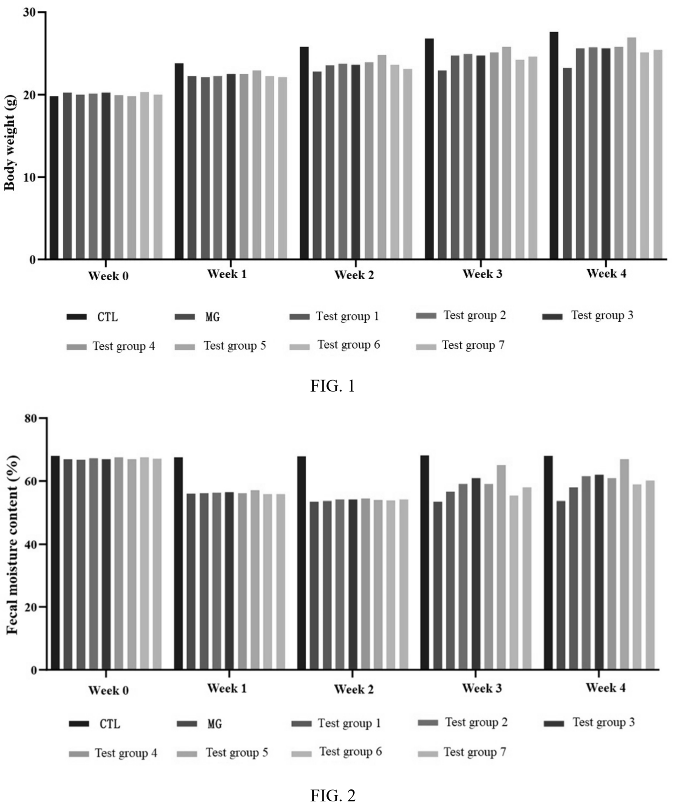

During the test, the body weights of mice were monitored every 7 days. After the test, the statistics on the body weights of the groups of mice are shown in FIG. 1. It can be seen from the figure that the body weight of mice in the model group increases slowly, and after the intervention of probiotics, the body weight of mice in test group 5 (BLa36+LRa05+BC99+BBi32) increases significantly, indicating that the composite probiotic of the present application can promote the growth of mice.

### (4) Analysis of fecal moisture contents of mice

During the test, mouse feces were collected. After collection, the wet weight of feces (A) was recorded, and after drying in an oven for 3 h, the dry weight of feces (B) was recorded. The moisture content was calculated as follows: fecal moisture content (%) = (A - B)/A × 100%. The results are shown in FIG. 2.

It can be seen from the figure that the fecal moisture content of mice in the control group remains stable at about 68%, the fecal moisture content of mice in the model group decreases rapidly and then remains stable at about 53.5% compared with that of the control group, and after two weeks of probiotic intervention, compared with that of mice in the model group, the fecal moisture contents of mice in the test groups increase to different degrees, and test group 5 (BLa36+LRa05+BC99+BBi32) exhibits the best effect, indicating that the composite probiotic of the present application can restore the intestinal functions.

### (5) Analysis of small intestinal propulsion rates of mice

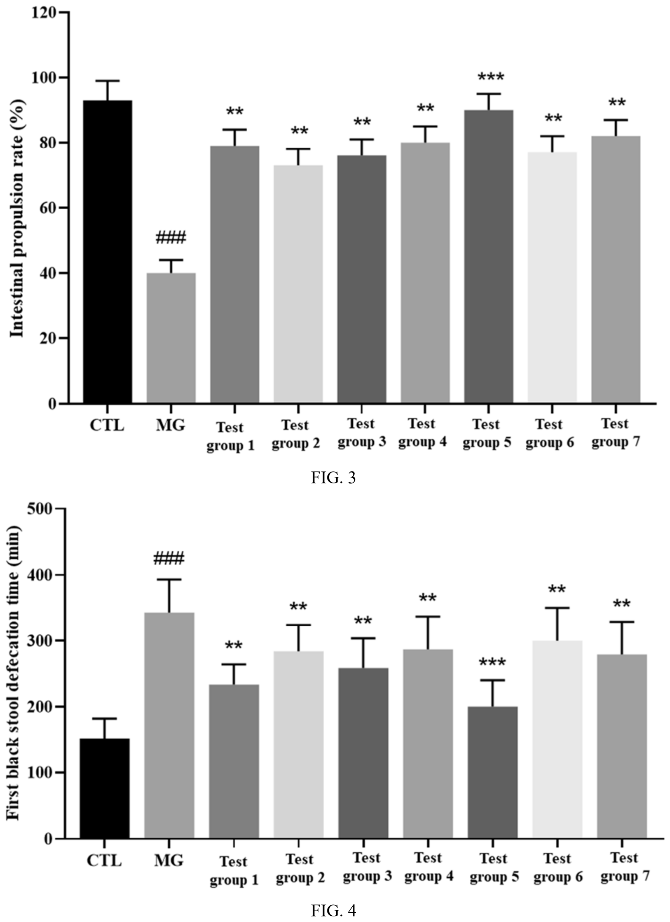

The intestinal transit rate (intestinal propulsion rate) was calculated as follows: intestinal transit rate (%) = C/D × 100% (where C denotes the length between the pyloric sphincter and the end of the charcoal-stained intestine, and D denotes the full length of the intestine (the distance from the pylorus to the rectum)). The results are shown in FIG. 3.

It can be seen from FIG. 3 that the intestinal propulsion rate of mice in the model group is significantly lower than that of the control group (p < 0.01), while the intestinal propulsion rates of mice after probiotic intervention are higher than that of the model group (p < 0.01), and test group 5 (BLa36+LRa05+BC99+BBi32) exhibits the best effect and has an intestinal propulsion rate close to that of the control group, further proving that the composite probiotic agent helps to restore the intestinal functions.

### (6) Analysis of first black stool defecation times of mice

During the test, the color of feces excreted by mice in each group was observed, the time when the black stools were excreted for the first time was recorded, and the time minus the time of gavage was the first black stool time of mice. The results are shown in FIG. 4.

The time for which the black stools pass through the whole intestinal tract in the constipated mice in the model group is significantly longer than that of the normal mice in the control group (p < 0.01). After probiotic intervention, the black stool defecation times of mice are significantly shorter (p < 0.01), and test group 5 (BLa36+LRa05+BC99+BBi32) exhibits the best improvement effect, further indicating that the composite probiotic have a significant effect in restoring the intestinal functions and can be applied to the preparation of a drug for preventing, alleviating, or treating constipation.

### (7) Analysis of short-chain fatty acid levels in feces of mice

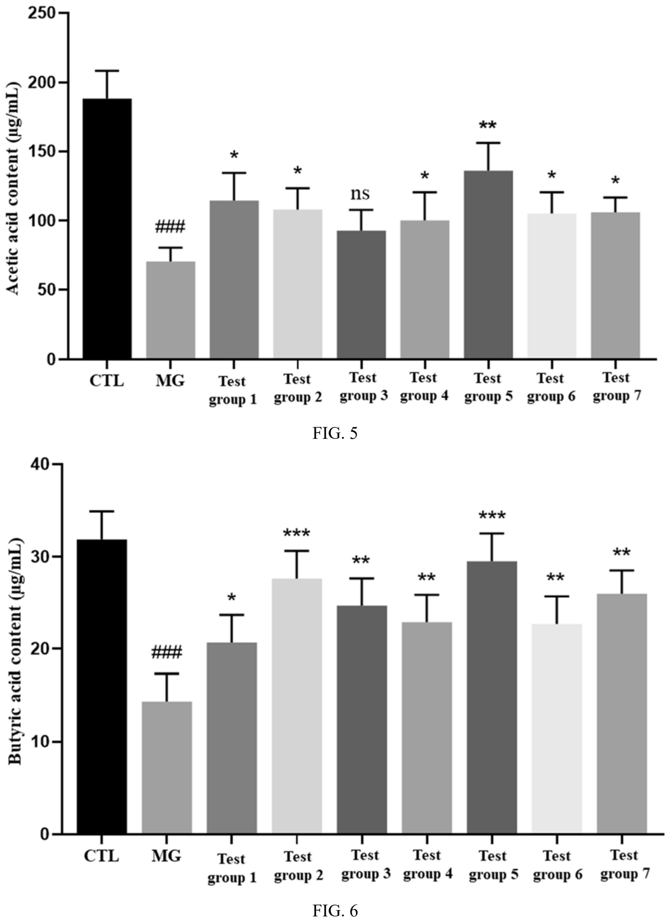

During the test, feces of mice in each group were collected during week 0 and week 4. The feces were vacuum-lyophilized. 25 mg of feces was sampled, immersed in 500 µL of saturated NaCl solution for 30 min, uniformly mixed through vortex shaking, acidified in 20 µL of 10% H₂SO₄ solution, extracted with 800 µL of anhydrous ether, and centrifuged at 18000 g for 15 min. The upper ether phase was dried over 250 mg of anhydrous Na₂SO₄ and centrifuged at 18000 g for 15 min. The upper ether phase was taken as a short-chain fatty acid mixture solution. The content of short-chain fatty acids (acetic acid and butyric acid) in the feces was analyzed through GC-MS by an external standard method. The results are shown in FIGS. 5 and 6.

It can be seen from FIGS. 5 and 6 that the contents of acetic acid and butyric acid in the feces of the constipated mice in the model group are significantly lower than those of the control group, while the contents of acetic acid and butyric acid in the feces of mice after probiotic intervention are increased to different degrees compared with those of the model group, and test group 5 (BLa36+LRa05+BC99+BBi32) exhibits the best effect, indicating that the composite probiotic can relieve constipation symptoms and regulate intestinal health by increasing the short-chain fatty acid level.

### (8) Analysis of gastrointestinal peptide levels in serum of mice

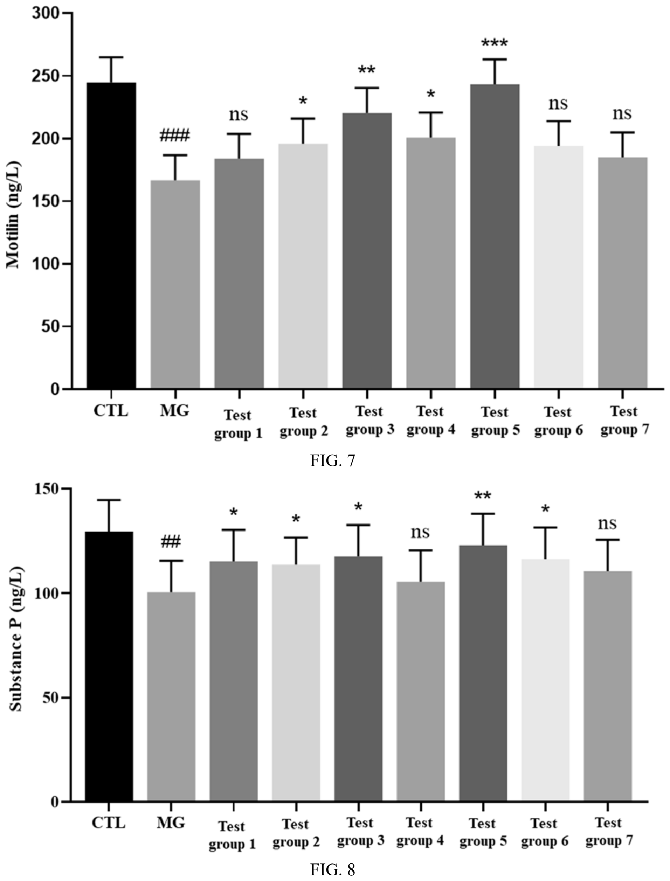

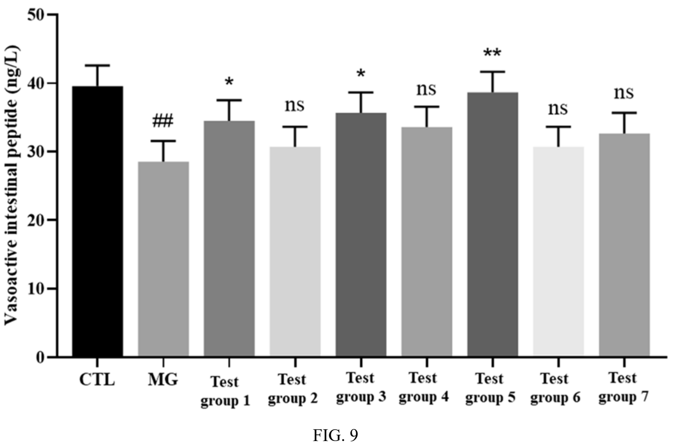

The concentrations of motilin (MTL), substance P (SP), and vasoactive intestinal peptide (VIP) in the serum of mice in each group were determined by an ELISA kit. The detection results are shown in FIGS. 7, 8, and 9, respectively.

The results show that the levels of motilin, substance P, and vasoactive intestinal peptide of the constipated mice in the model group are significantly lower than those of the normal mice in the control group, while the levels of motilin, substance P, and vasoactive intestinal peptide of mice after probiotic intervention are increased to different degrees compared with those of the constipated mice in the model group, and test group 5 (BLa36+LRa05+BC99+BBi32) exhibits the best effect and the levels are close to the normal values, proving that the composite probiotic can restore the normal intestinal peristalsis and relieve constipation symptoms by regulating the level of gastrointestinal peptide hormones.

In summary, the composite probiotic of the present application have good efficacy in improving constipation symptoms, and the BLa36 strain, the LRa05 strain, the BC99 strain, and the BBi32 strain can collaborate with each other, promote each other, and achieve a synergistic effect in promoting intestinal peristalsis and relieving constipation symptoms.

The applicant has stated that although the technical solutions of the present application are described through the preceding embodiments, the present application is not limited to the preceding embodiments, which means that the implementation of the present application does not necessarily depend on the preceding embodiments. It is to be understood by those skilled in the art that any improvements made to the present application and equivalent replacements of raw materials of the product, additions of adjuvant ingredients, selections of specific manners, etc. in the present application all fall within the scope of the present application.

Although preferred embodiments of the present application are described above in detail, the present application is not limited to details of the preceding embodiments, and various simple variations can be made to the technical solutions of the present application without departing from the technical concept of the present application. These simple variations are all within the scope of the present application.

Additionally, it is to be noted that if not in collision, specific technical features described in the preceding embodiments can be combined in any suitable manner. To avoid unnecessary repetition, various possible combinations are no longer described in the present application.

## Claims

1. A composite probiotic for improving constipation, which is composed of a *Bifidobacterium animalis subsp. lactis* BLa36 strain deposited under CGMCC No. 24029, a *Lactobacillus rhamnosus* LRa05 strain deposited under CGMCC No. 24377, a *Bacillus coagulans* BC99 strain deposited under CGMCC No. 19487, and a *Bifidobacterium bifidum* BBi32 strain deposited under CGMCC No. 16923.

2. The composite probiotic for improving constipation according to claim 1, wherein a ratio of a viable bacteria count of the BLa36 strain, a viable bacteria count of the LRa05 strain, a viable bacteria count of the BC99 strain, and a viable bacteria count of the BBi32 strain is (1-10):(1-10):(1-10):(1-10).

3. A probiotic agent for improving constipation, comprising the composite probiotic according to claim 1 or 2.

4. The probiotic agent for improving constipation according to claim 3, wherein a viable bacteria count of each of the BLa36 strain, the LRa05 strain, the BC99 strain, and the BBi32 strain in the probiotic agent is not less than 1×10⁸ CFU/mL or 1×10⁸ CFU/g.

5. The probiotic agent for improving constipation according to claim 3, wherein a dosage form of the probiotic agent comprises a solution, lyophilized powder, a capsule, a tablet, or a granule.

6. The probiotic agent for improving constipation according to claim 3, wherein the probiotic agent further comprises a lyoprotectant and/or an auxiliary additive.

7. The probiotic agent for improving constipation according to claim 6, wherein the lyoprotectant comprises any one or a combination of at least two of skimmed milk, sucrose, lactose, trehalose, dextran, gelatin, dextrin, arabic gum, sodium alginate, polyvinylpyrrolidone, sorbitol, or xylitol.

8. A use of the composite probiotic according to claim 1 or 2 or the probiotic agent according to any one of claims 3 to 7 for preparing a drug for preventing, alleviating, or treating constipation.

9. A use of the composite probiotic according to claim 1 or 2 or the probiotic agent according to any one of claims 3 to 7 for preparing a drug for increasing a gastrointestinal peptide level.

10. A use of the composite probiotic according to claim 1 or 2 or the probiotic agent according to any one of claims 3 to 7 for preparing a drug for increasing a short-chain fatty acid level in a gastrointestinal tract.
